# EUROPEAN PATENT APPLICATION

(11) **EP 1 046 404 A2**
(43) Date of publication of application: **25.10.2000**
(21) Application number: 00201394.4
(22) Date of filing: 19.04.2000
(51) Int. Cl.: A61M 5/32

(54) **Sharps destroyer**

(30) Priority: 21.04.1999 GB 9909087
(71) Applicant: Sharpaway Limited, Londom SE1 1HH (GB)
(72) Inventor: Clarke, Howard Morgan, Liss, Hampshire GU33 7NT (GB)
(74) Representative: Brooks, Nigel Samuel

(57) **Abstract**

A device (1) for destroying needles and the like, comprising a body (3) holding batteries and a charging circuit and a disposable head (2) housing the electrodes (41, 42) and an aperture (22) in its top to provide access to the electrodes. The electrodes are shaped to allow a needle inserted in the aperture to come into contact with both electrodes. The electrodes are made from copper, either in combination with carbon, or with carbon coated onto at least the under surface of the upper electrode. This prevents fragments of the needle from adhering to the copper electrodes as it is destroyed and arcing, causing pitting of the electrodes. The charging circuit allows a rapid charge of the batteries and a test circuit is provided which allows and operator to check whether there is enough charge for the destruction of a needle or whether the batteries need to be charged.

## Description

The present invention relates to apparatus for destroying needles in particular hypodermic needles.

The disposal of needles and the like causes a serious problem. Frequently the needle contains traces of blood products or other hazardous materials. Such needles must be disposed of in such a way that there is no danger to the disposer by way of stabbing by the needle. Thus these needles cannot be placed in a standard plastic refuse sack as the needles will pierce the side of the sack and can injure a handler. One method commonly used is to provide a separate box for these needles. The boxes are usually made of stiffened plastic material and have a small aperture in their top through the needle can be pushed. However, although this reduces the likelihood of injury to another person, it does not destroy any toxic residue which may be present on the needle. Furthermore, for health workers who may have to travel to see their patients it is not very convenient nor safe to carry around a box containing the needles used on previous patients.

Another method of destroying these needles is to incinerate them. This destroys the toxic residue on the needle as well as the needle itself. It is known to provide incinerators as portable hand held devices. The needles are pushed through a small aperture in the device onto a pair of electrodes held within the device. A voltage is held across the electrodes which on contact with the needle passes a current through the needle high enough to melt and at least partially burn the needle. However, this type of device has not been very reliable as during the destruction of the needle, small fragments of the molten or partially destroyed needle may adhere to the electrodes causing arcing and bridging of the electrodes. This wastes energy stored in batteries, and causes pitting of the electrodes, wearing them out very rapidly.

The object of the present invention is to provide an improved device for destroying needles.

According to the invention there is provided an electrode device for destroying metallic sharps, the device comprising
- a pair of spaced electrodes; and
- means for passing an electrical current therebetween for destruction of a metallic sharp by ohmic heating when the sharp extends into a gap between the electrodes
characterised in that at least one of the electrodes has a surface at the gap which includes non-metallic material for discouraging adhesion of molten sharps debris thereto.

Preferably the non-metallic material is included on an underside of an overhanging one of the electrodes. Normally the non-metallic material will be carbon, and the electrodes will be made of or include copper.

The electrodes may be made from heat hardened copper and coated on at least one surface with graphite having a thickness of approximately 5 microns (µm).

However, normally at least one electrode will have a surface at the gap which is made from a sintered mixture including copper and carbon. Typically the quantity of copper in the sintered mixture is approximately 82-85% and the quantity of carbon is approximately 12%, and the other material in the sintered mixture comprises binding agents, tin and zinc.

According to a second aspect of the invention there is provided equipment for destroying metallic sharps, characterised in that the equipment comprises a body and a disposable head, the body and the head having co-operating mating parts and contacts for mechanical and electrical connection;
- the body having a casing containing
   - electrical current supply means including batteries and a charging circuit therefor;
- the disposable head having a casing containing
   - an electrode device of the first aspect of the invention;
   - an aperture in the casing above the electrodes, for access of a metallic sharp to the electrodes.

Preferably the charging circuit is adapted to charge from a car battery or any 12v source.

Preferably the charging circuit includes a switch mode device for charging the batteries, and monitoring means for monitoring the charge level in batteries. More particularly the monitoring means is a DS 1223.

To help understanding of the invention, a specific embodiment thereof will now be described by way of example and with reference to the accompanying drawings in which:-
Figure 1 is a top view of the device;
Figure 2 is a front perspective view of the body;
Figure 3 is a front perspective view of the disposable head;

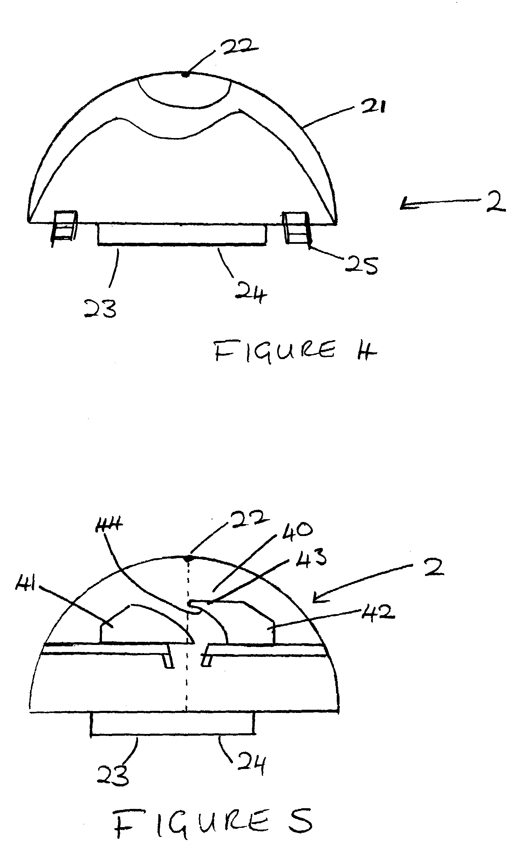

Figure 4 is a front view of the head; and
Figure 5 is a side view of the electrodes;

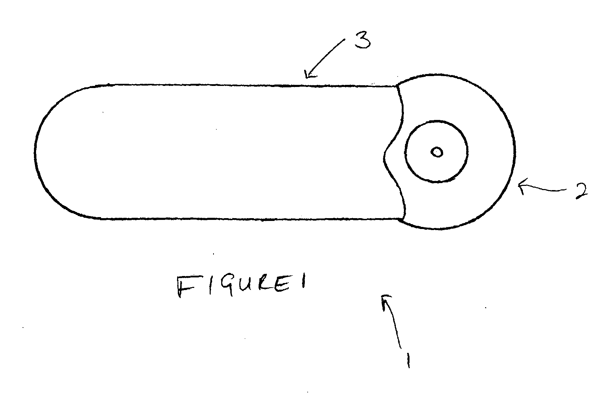

Referring to Figure 1, the device 1 is divided into two parts; a head 2 and a body 3, designed in use to be connected to each other. The head 2 is domed 21 having an aperture 22 in its top. The head 2 has a flat underside 23 with an rectangular extension 24 along its length. The body 3 is rectangular in shape having extensions 31 at one end enabling the extension 24 of the head 2 to fit between them. The head 2 and body 3 have complimentarily shaped electrical connections 25, 32.

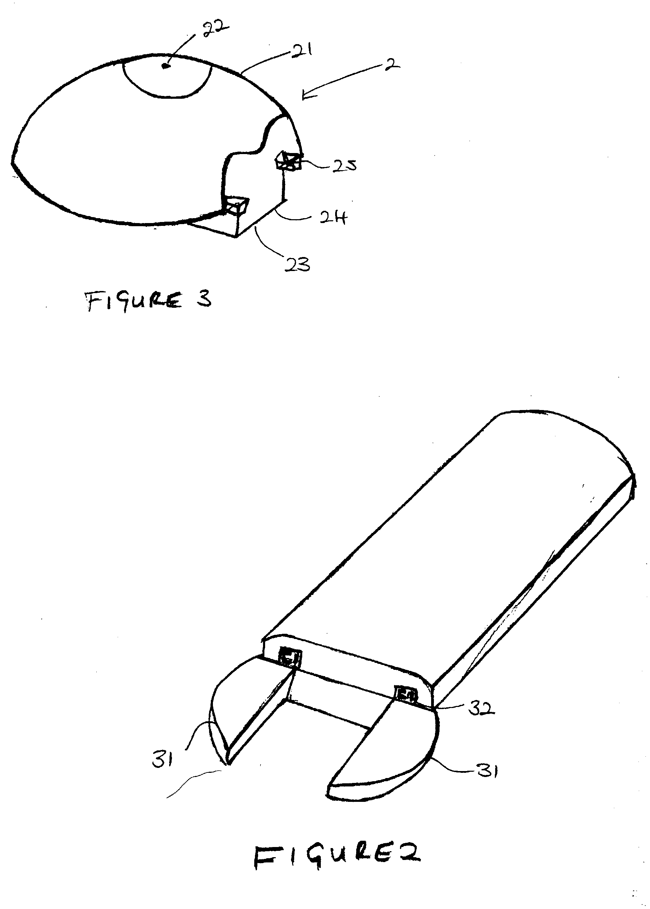

Referring to Figure 3, the body 3 houses a charging circuit. The circuit is designed to charge from a car battery or any 12 v source, over a period of 4 hours. Each charge is designed to destroy 30 needles. The charging circuit includes an indicator to show that the battery holds enough power to destroy a needle.

The charging circuit includes a standard switch mode device, or DC to DC switching controller for charging standard 4v batteries. The switch mode device allows maximum power to be fed to the batteries without high heat loss. The charging circuit also includes means for monitoring the charge level of the batteries. A DS 1223 is used to monitor the voltage level. This standard device is usually used in a low-voltage monitoring device, where it monitors the voltage, and if it falls below a specified level, the device will switch the monitor into re-start mode. In the present invention the device is used to monitor the charge level in the batteries. Upon depression of a test switch the device is connected across the batteries. If the charge level is above a minimum, a green led is activated, while if the charge level is on or below the minimum a red led is activated indicating that the batteries need recharging. Approximately thirty needles can be destroyed on a single charge.

Referring to Figure 5, the head 2 holds a pair of electrodes 40, directly beneath the aperture 22. The electrodes are shaped so that a needle or the like pushed into the aperture will contact both electrodes. One of the electrodes, 41 is approximately domed in shape, to allow a needle a large surface area to contact. The other electrode 42 is shaped rather like an anvil, with the extension 43 above at least a part of the domed lower electrode 41. Thus a needled inserted through the aperture 22 in the domed head 21, with contact the edge of the extension 43 of the anvil shaped electrode 42, and the domed electrode 41, resulting, when the device is switched on, in a current passing through the needle.

Each electrode is made from heat hardened copper and coated in a thin layer of graphite, of the order of 5 microns thick. While it has been found that it is only necessary to coat the underside 44 of the upper electrode in graphite, in practice, the whole electrode is usually graphite coated. For maximising the life of the electrodes both are graphite coated. Alternatively one or both the electrodes are made from a sintered mixture of copper and graphite. A typical mixture would contain approximately 82-85% copper and approximately 12% graphite, together with small quantities of a binding agent, tin and zinc.

Electrodes made purely of copper become pitted as small fragments of the molten needle adheres to its surface, resulting in arcing and/or bridging to the other electrode. However the presence of carbon in the electrodes, either as a coating or integral part of the electrode greatly reduces this. It is particularly important to ensure that the lower surface 43 of the upper electrode 42 includes carbon as this is particularly prone to damage due to adherence of debris produced by spitting as the needle comes into contact with the lower electrode. Any debris build up on this section of the electrode reduces the distance between the electrodes, and hence the likelihood of arcing or bridging. With the addition of carbon as coating or constituent of the electrode, as the molten swarf from the needle moves between the electrodes it continues to melt without arcing and then falls away from the electrodes. The use of copper in the electrodes allows a high current to be used, of the order of 400 amps. Copper also has a high heat capacity, resulting in low impedance in the electrodes when a current is passed through them. The use of copper insures that the heat capacity of the electrodes is higher than that of the needles preventing over heating of the electrodes. Thus the current flowing through the needle melts and burns it, but does not adversely effect the electrodes. If the electrodes are composed of a mixture of carbon and copper, the amount of the carbon is critical to ensure that these electrical properties are not compromised, while the presence of the carbon prevents damage to the electrodes during the destruction of the needles.

The electrodes are shaped so that a needle introduced into the aperture can contact both electrodes. The shape of the electrodes is also important for allowing the debris from the melting and burning to fall away into the compartment. The positioning of the electrodes ensures that the device operates efficiently. If the separation is too large, then a larger current would be necessary to overcome the resistance in the needle and thick needles may not be destroyed. On the other hand if the separation is too small, the debris from the melting of the needles can cause bridging between the electrodes leading to discharge from the battery. Typically the separation between the needles will be between 1-5 and 2.0 mm in either direction.

## Claims

1. An electrode device (2) for destroying metallic sharps, the device comprising
• a pair of spaced electrodes (41, 42); and
• means (25) for passing an electrical current therebetween for destruction of a metallic sharp by ohmic heating when the sharp extends into a gap between the electrodes
characterised in that at least one of the electrodes (42) has a surface (44) at the gap which includes non-metallic material for discouraging adhesion of molten sharps debris thereto.

2. An electrode device (2) for destroying metallic sharps as claimed in claim 1, characterised in that the non-metallic material is included on an underside (44) of an overhanging one of the electrodes (42).

3. An electrode device (2) for destroying metallic sharps as claimed in claim 1 or claim 2, characterised in that the non-metallic material is carbon.

4. An electrode device (2) for destroying metallic sharps as claimed in claim 1, claim 2 or claim 3, characterised in that the electrodes (41, 42) are made of copper or include copper.

5. An electrode device (2) for destroying metallic sharps as claimed in any one of claims 1 to 4, characterised in that the electrodes (41, 42) are made from heat hardened copper and at least one surface (44) of which is coated in graphite.

6. An electrode device (2) for destroying metallic sharps as claimed in claim 5, characterised in that the thickness of the graphite coating is of the order of 5 microns (µm).

7. An electrode device (2) for destroying metallic sharps as claimed in any one of claims 1 to 4, characterised in that at least one electrode (42) has a surface (44) at the gap which is made from a sintered mixture including copper and carbon.

8. An electrode device (2) for destroying metallic sharps as claimed in claim 6, characterised in that the quantity of copper in the electrode (42) is approximately 82-85% and the quantity of carbon is approximately 12%.

9. An electrode device (2) for destroying metallic sharps as claimed in claim 8 characterised in that that the other material in the electrode (42) comprises binding agents, tin and zinc.

10. Equipment for destroying metallic sharps (1), characterised in that the equipment comprises a body (3) and a disposable head (2), the body (3) and the head (2) having co-operating mating parts (23, 31) and contacts (25, 32) for mechanical and electrical connection;
• the body (3) having a casing containing
• electrical current supply means including batteries and a charging circuit therefor;
• disposable head (2) having a casing (21) containing
• an electrode device (2) as claimed in any preceding claim.
• an aperture (22) in the casing (21) above the electrodes (41, 42), for access of a metallic sharp to the electrodes.

11. Equipment for destroying metallic sharps (1) as claimed in claim 10, characterised in that the charging circuit is adapted to charge from a car battery or any 12v source.

12. Equipment for destroying metallic sharps (1) as claimed in claim 10 or claim 11, characterised in that the charging circuit includes a switch mode device for charging the batteries, and monitoring means for monitoring the charge level in batteries.

13. Equipment for destroying metallic sharps (1) as claimed in claim 12, characterised in that the monitoring means is a DS 1223.
